# EUROPEAN PATENT APPLICATION

(11) **EP 4 368 121 A1**
(43) Date of publication of application: **15.05.2024**
(21) Application number: 22855195.8
(22) Date of filing: 20.07.2022
(51) Int. Cl.: A61B 17/00

(54) **HANDLE, CONVEYOR, AND MEDICAL DEVICE**

(30) Priority: 09.08.2021 CN 202110910117
(71) Applicant: Shanghai Microport Cardioflow Medtech Co., Ltd., Shanghai 201203 (CN)
(72) Inventor: LIU, Shihong, Shanghai 201203 (CN); LIN, Xing, Shanghai 201203 (CN); JI, Lijun, Shanghai 201203 (CN); CHEN, Guoming, Shanghai 201203 (CN)
(74) Representative: Marks & Clerk LLP
(86) International application number: PCT/CN2022/106786
(87) International publication number: WO 2023/016212

(57) **Abstract**

The present invention provides a handle, a conveyor and a medical device. The conveyor includes a handle configured to control a catheter assembly. The catheter assembly includes an outer tube and an inner tube inserted in the outer tube. The outer tube or the inner tube is used as a first movable tube. The handle includes a housing; a first operating part configured to drive the first movable tube to move in a first direction; a first connecting component connected in the housing; a second connecting component connected to the first operating part; when the second connecting component is connected to the first connecting component, the first operating part is prevented from driving the first movable tube to move in the first direction, and when at least one of the second connecting component and the first connecting component rotates to release the connection, the first operating part is allowed to drive the first movable tube to move. After the release of the medical device is completed by using the conveyor to control the catheter assembly, the connection between the second connecting component and the first connecting component is released by rotating the second connecting component, and the first operating part is operated to close the inner tube and the outer tube to meet the usage requirements.

## Description

### TECHNICAL FIELD

The present invention relates to the technical field of medical instruments and, more specifically, to a handle, a conveyor and a medical device.

### BACKGROUND

In recent years, the prevalence of coronary heart disease, cardiovascular and cerebrovascular diseases, heart diseases, tumors and other diseases among middle-aged and elderly people has increased year by year. These diseases directly affect the quality of life and even life safety of middle-aged and elderly people. At present, traditional surgery is still the preferred treatment for this type of disease. However, for patients who are elderly, have multiple organ diseases, have a history of thoracotomy, and have poor physical recovery function, traditional surgery has high risks and high mortality. There was not even a chance for some patients to receive a surgery.

Interventional surgery is a brand-new treatment technology developed in recent years and is a minimally invasive surgical treatment. Interventional therapy is for introduction of special medical devices into the human body through a conveyor under the guidance of medical imaging equipment to diagnose and locally treat lesions in the body. Interventional treatment does not require surgery. Compared with traditional surgery, it has the advantages of less trauma, faster recovery, and better results. For specific diseases, the medical devices introduced into the human body for interventional treatment are different. For example, for heart valve disease, the medical device can be a valve prosthesis. For other vascular diseases, the medical device can be a medical stent or embolization spring coil or others.

The conveyor usually includes a handle and a catheter assembly. The catheter assembly is used to load medical devices and includes an inner tube and an outer tube. The handle is used to connect with the catheter assembly and control the inner tube and outer tube to enable the medical device to be introduced into human body. As the power source during the entire interventional surgery, the handle needs to ensure sufficient safety, effectiveness and economy. There are different requirements for the operation of the handle at different stages of interventional surgery. For example, in valve prosthesis implantation, when positioning the valve prosthesis, the handle is required to control the catheter assembly to release the valve prosthesis as slowly as possible. After the positioning of the valve prosthesis is completed, in order to avoid excessive reduction in blood pressure, the handle is required to control the catheter assembly to release the valve as quickly as possible, and after the valve is completely released, in order to reduce the closing time of the inner tube and the outer tube, the handle is required to be able to control the inner tube to move back quickly.

### SUMMARY OF THE INVENTION

An object of the present invention is to provide a handle, a conveyor and a medical device, which can properly control the catheter assembly in interventional surgery according to the actual situation to meet the operation requirements.

In order to achieve the above object, the present invention provides a handle, for controlling a catheter assembly, the catheter assembly comprising an outer tube and an inner tube partially inserted in the outer tube, the outer tube or the inner tube used a first movable tube; wherein the handle comprises:
a housing;
a first operating part, connected to the housing, and configured to drive the first movable tube to move in a first direction;
a first connecting component connected in the housing; and
a second connecting component connected to the first operating part, and the second connecting component selectively connected to or disconnected from the first connecting component;
wherein the first connecting component and/or the second connecting component are configured to be rotatable about an axis of the first movable tube; and the handle is configured such that, when the second connecting component and the first connecting component are connected with each other, the first operating part is prevented from driving the first movable tube to move in the first direction, when the first connecting component and/or the second connecting component rotate around the axis of the first movable tube to cause the first connecting component and the second connecting component to disconnect from each other, the first operating part is allowed to drive the first movable tube to move in the first direction.

Optionally, the inner tube is used as the first movable tube, and the first direction is a direction from distal to proximal.

Optionally, the first connecting component comprises a connector tube and a first limiting part, wherein the connector tube is connected to the housing and is configured for allowing the first movable tube to pass through; the first limiting part is connected to the connector tube and extends outward along a radial direction of the connector tube;
wherein the second connecting component comprises a second positioning seat, wherein the second positioning seat is connected to an inner wall of the first operating part and is configured to be rotatable around an axis of the first operating part; the second positioning seat defines therein a first central through hole and an avoidance slot, the avoidance slot is connected with the first central through hole;
wherein the handle is configured such that, when the connector tube passes through the first central through hole, and when the first limiting part is staggered with the avoidance slot in a circumferential direction and pressed against the second positioning seat, the second connecting component and the first connecting component are connected to each other, when the second positioning seat rotates until the avoidance slot aligns with the first limiting part in the circumferential direction and allows the first limiting part to pass through, the second connecting component and the first connecting component are disconnected from each other.

Optionally, a guide surface is formed on the first limiting part, a distance between the guide surface and an axis of the connector tube gradually increases along the first direction, and the guide surface is configured for guidance when the first limiting part passes through the avoidance slot.

Optionally, the first operating part defines thereon a first slide slot extending along a circumferential direction of the first operating part; the second connecting component further comprises a first lever having one end connected to the second positioning seat and a further end extending out of the first operating part through the first slide slot.

Optionally, the second connecting component further comprises a first elastic sleeve sleeved on the first lever, and an outer wall of the first elastic sleeve is in contact with a wall of the first slide slot so that the first elastic sleeve deforms.

Optionally, the first connecting component further comprises a first positioning seat, and the connector tube is connected to the housing by the first positioning seat; the first positioning seat comprises a first positioning sub-seat, a connecting arm and a second positioning sub-seat that are sequentially connected along the first direction; the first positioning sub-seat is connected to the housing; the second positioning sub-seat is connected to the connector tube.

Optionally, a number of the connecting arms is at least two, and the at least two connecting arms are arranged at intervals along a circumference of the first positioning sub-seat.

Optionally, when the inner tube is used as the first movable tube, the first direction is a direction from distal to proximal, and the first operating part is arranged proximally to the housing;
the handle further comprises an auxiliary connecting plate that is sleeved on the connector tube and pressed against a proximal end face of the second positioning sub-seat;
when the first connecting component and the second connecting component are connected with each other, a distal end of the first operating part is pressed against a proximal end face of the auxiliary connecting plate.

Optionally, when the inner tube is used as the first movable tube, the handle further comprises a fourth connecting component that is connected to the inner wall of the first operating part and also configured to connect with a proximal end of the inner tube.

Optionally, the fourth connecting component comprises an anti-rotation member having a second central through hole for allowing the inner tube to pass through; the anti-rotation member comprises a first anti-rotation part and a second anti-rotation part; the first anti-rotation part is connected to the first operating part and is configured to remain circumferentially stationary relative to the first operating part; the second anti-rotation part is at least partially inserted into an inner cavity of the connector tube and is configured to remain circumferentially stationary relative to the connector tube.

In order to achieve the above object, the present invention also provides a conveyor, comprising a catheter assembly and the above handle, wherein the catheter assembly comprises an outer tube and an inner tube partially inserted in the outer tube, and the outer tube or the inner tube is used a first movable tube; the first operating part of the handle is connected to the first movable tube and is configured to drive the first movable tube to move along the first direction.

In order to achieve the above object, the present invention also provides a medical device, comprising the above conveyor and a medical implant, wherein the medical implant is configured to be compressed between the inner tube and the outer tube.

Compared with the prior art, the handle, conveyor and medical device of the present invention have the following advantages:
The conveyor comprises a catheter assembly and a handle, wherein the catheter assembly comprises an outer tube and an inner tube partially inserted in the outer tube, and the outer tube or the inner tube is used a first movable tube. The handle comprises a housing, a first operating part, a first connecting component and a second connecting component. The first operating part is connected to the housing and configured under an action of external force to drive the first movable tube to move in a first direction. The first connecting component is connected in the housing. The second connecting component is connected to the first operating part, and the second connecting component is selectively connected to the first connecting component. The first connecting component and/or the second connecting component are configured to be rotatable about an axis of the first movable tube; and the handle is configured such that, when the second connecting component and the first connecting component are connected with each other, the first operating part is prevented from driving the first movable tube to move axially, when the first connecting component and/or the second connecting component rotate around the axis of the first movable tube to cause the first connecting component and the second connecting component to disconnect from each other, the first operating part is allowed to drive the first movable tube to move in the first direction. When the inner tube is used as the first movable tube, the first direction is a direction from distal to proximal. When the outer tube is used as the first movable tube, the first direction is a direction from proximal to distal. In this way, when the medical implant is implanted into the patient's body using the conveyor, and when the inner tube needs to be retracted into the outer tube, the second connecting component can be rotated to be disconnected from the first connecting component, and then the first operating part is operated to quickly retract the inner tube into the outer tube.

Further, the first connecting component comprises a connector tube and a first limiting part. The connector tube is connected to the first positioning seat and is used for allowing the first movable tube to pass through. The first limiting part is connected to the connector tube and extends outward in the radial direction of the connector tube. The second connecting component comprises a second positioning seat, the second positioning seat is connected to the second operating part and is configured to be rotatable around the axis of the first operating part. The second positioning seat defines therein a first central through hole and an avoidance slot. The first central through hole is configured for allowing the connector tube to pass through. The avoidance slot is connected with the first central through hole and is configured for allowing the first limiting part to pass through. When the connector tube passes through the first central through hole, and when the first limiting part is staggered with the avoidance slot in the circumferential direction and pressed against the second positioning seat, the second connecting component and the first connecting component are connected with each other. When the second positioning seat rotates until the avoidance slot aligns with the first limiting part in the circumferential direction and allows the first limiting part to pass through, the second connecting component and the first connecting component are disconnected from each other. That is to say, the first connecting component and the second positioning seat can be connected or disconnected by rotating the second positioning seat, and then the first operating part drives the first movable tube to move. The operation is simple with less error.

### BRIEF DESCRIPTION OF THE DRAWINGS

The present invention will be better understood with reference to the accompanying drawings provided without limiting the invention, in which:
Fig. 1 is a structural schematic diagram of a conveyor according to an embodiment of the present invention;
Fig. 2 is a partial structural schematic diagram of a conveyor according to an embodiment of the present invention;
Fig. 3 is a partial structural schematic diagram of a conveyor according to an embodiment of the present invention, in which only part of the housing is shown;
Fig. 4 is a partial enlarged view of the handle shown in Fig. 3;
Fig. 5 is a structural schematic diagram of the first connecting component of the handle of the conveyor according to an embodiment of the present invention;
Fig. 6 is a partial enlarged view of the first connecting component shown in Fig. 5;
Fig. 7 is a structural schematic diagram of a first connecting component of a handle of a conveyor according to an embodiment of the present invention, which is viewed from a perspective different from that of Fig. 5;
Fig. 8 is a partial enlarged view of the first connecting component shown in Fig. 7;
Fig. 9 is a schematic diagram of the proximal end face of the connector tube of the handle of the conveyor according to an embodiment of the present invention;
Fig. 10 is a structural schematic diagram of the second positioning seat of the handle of the conveyor according to an embodiment of the present invention;
Fig. 11 is a structural schematic diagram of the second positioning seat of the handle of the conveyor according to an embodiment of the present invention, which is viewed from a perspective different from that of Fig. 10;
Fig. 12 is a structural schematic diagram of the auxiliary connecting plate of the handle of the conveyor according to an embodiment of the present invention;
Fig. 13 is a structural schematic diagram of the anti-rotation member of the handle of the conveyor according to an embodiment of the present invention.

List of reference numerals:
1000, handle; 1110, housing; 1111, third slide slot; 1112, observation window; 1120, first operating part; 1121, first slide slot; 1130, second operating part; 1101, first fastening ring; 1102, second fastening ring; 1210, first positioning seat; 1211, first positioning sub-seat; 1212, connecting arm; 1213, second positioning sub-seat; 1220, connector tube; 1230, first limiting part; 1231, guide surface; 1310, second positioning seat; 1311, first central through hole; 1312, avoidance slot; 1320, first lever; 1330, first elastic sleeve; 1400, auxiliary connecting plate; 1410, second slide slot; 1510, screw rod; 1520, threaded sleeve; 1610, first limiting structure; 1611, third positioning seat; 1612, second limiting part; 1613, second lever; 1614, second elastic sleeve; 1620, second limiting structure; 1621, third limiting part; 1710, anti-rotation member; 1711, second central through hole; 1712, first anti-rotation part; 1713, second anti-rotation part;
2100, outer tube; 2200, inner tube.

### DETAILED DESCRIPTION

The following describes the embodiments of the present invention through specific examples. Those skilled in the art can easily understand other advantages and effects of the present invention from the content disclosed in this specification. The present invention can also be implemented or applied through other different specific embodiments. Various details in this specification can also be modified or changed in various ways based on different viewpoints and applications without departing from the spirit of the present invention. It should be noted that the diagrams provided in this embodiment only illustrate the basic concept of the present invention in a schematic manner. The drawings only show the components related to the present invention and do not follow the numbers, shapes and dimensions of components in actual implementation, the type, quantity and proportion of each component can be arbitrarily changed, and arrangement of component may also be more complex.

In addition, each embodiment described below has one or more technical features, but this does not mean that the inventor must implement all the technical features in any embodiment at the same time, or can only implement all or some of the technical features in different embodiments separately. In other words, on the premise that implementation is possible, those skilled in the art can, based on the disclosure of the present invention and design specifications or implementation requirements, selectively implement some or all of the technical features in any embodiment, or selectively implement a combination of some or all of the technical features in multiple embodiments, thereby increasing the flexibility of the implementation of the present invention.

As used in this specification, the singular forms "a", "an" and "the" include plural referents, and the plural form "plurality" includes two or more referents unless the content clearly dictates otherwise. As used in this specification, the term "or" is generally used in its sense including "and/or" unless the content clearly dictates otherwise, and the terms "mounted," "connected," and "connected" shall be understood in a broad sense, for example, it can be a fixed connection, a detachable connection, or an integral connection. The connection can be mechanical or electrical. It can be a direct connection or an indirect connection through an intermediary. It can be an internal connection between two elements or an interaction between two elements. For those of ordinary skill in the art, the specific meanings of the above terms in the present invention can be understood according to specific circumstances.

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

As used herein, the terms "proximal" and "distal" refer to the relative orientation, relative position, and direction of elements or actions relative to each other from the perspective of a doctor using the medical device. Although "proximal" and "distal" are not restrictive, "proximal end" usually refers to the end of the medical device that is close to the doctor during normal operation, and "distal end" usually refers to the end that first enters the patient's body, that is, the "proximal end" is the end far away from the patient, and the "distal end" is the end close to the patient. The term "axial" refers to the direction coincident with or parallel to the central axis of the medical device; "radial" refers to the direction perpendicular to the central axis of the medical device.

The embodiment according to the present invention provides a conveyor for conveying and releasing a medical implant to a predetermined position in a patient's body. The medical implant can be but not limited to a medical stent, valve prosthesis or embolization spring coil.

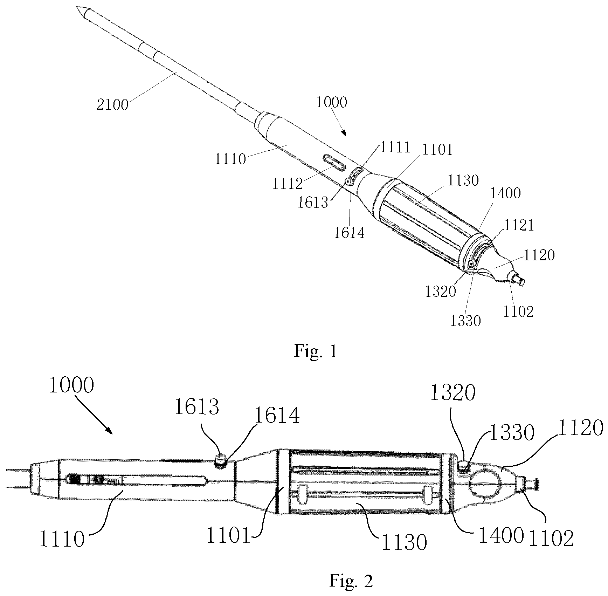

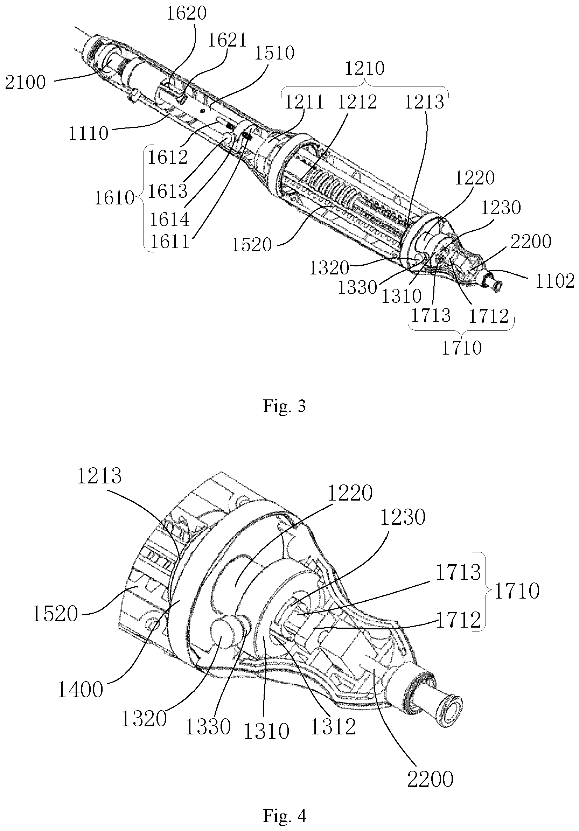

Fig. 1 shows a schematic diagram showing the overall structure of the conveyor, Fig. 2 shows a schematic diagram showing the structural appearance of the handle 1000 of the conveyor, and Fig. 3 shows a structural schematic diagram of the handle 1000 with part of the housing omitted.

Please refer to Figs. 1 to 3, the conveyor includes a handle 1000 and a catheter assembly. The catheter assembly includes an outer tube 2100 and an inner tube 2200 partially inserted in the outer tube 2100. The outer tube 2100 or the inner tubes 2200 is used as a first movable tube. The medical implant is loaded on the outer surface of the inner tube 2200 and compressed between the inner tube 2200 and the outer tube 2100. After it is implanted in the patient's body and the release of the medical implant is completed, the distal end of the inner tube 2200 protrudes from the distal end of the outer tube 2100. The handle 1000 is required to be used to control the first movable tube to move in a first direction so that the distal end of the inner tube 2200 moves back into the outer tube 2100. Therefore, the inner tube 2200 and the outer tube 2100 are closed. It can be understood that, when the distal end of the inner tube 2200 is provided with a tapered tip, and when the inner tube 2200 and the outer tube 2100 are closed, the tapered tip is located outside the distal end of the outer tube 2100. In an optional implementation, the inner tube 2200 is used as the first movable tube, and the first direction is a direction from distal to proximal. In this case, the inner tube 2200 and the outer tube 2100 can be closed by moving the inner tube 2200 in the direction from distal to proximal (i.e., by moving back the inner tube 2200). In another optional implementation, the outer tube 2100 is used as the first movable tube, and the first direction is a direction from proximal to distal. In this case, the inner tube 2200 and the outer tube 2100 can be closed by moving the outer tube 2100 in the direction from proximal to distal (i.e., by push forward the inner tube 2200). For ease of understanding, the following description takes the inner tube 2200 as the first movable tube as an example, but this should not unduly limit the present invention. Those skilled in the art can modify the following description to adapt it to the circumstance where the outer tube 2100 is used as the first movable tube.

Specifically, the handle 1000 includes a housing 1110, a first operating part 1120, a first connecting component and a second connecting component. The housing 1110 can be used for the operator to hold. The first operating part 1120 is preferably movably connected to the housing 1110 and configured to drive the inner tube 2200 along the direction from distal to proximal under the action of external force. The first connecting component is connected in the housing 1110. The second connecting component is partially disposed in the housing and connected to the first operating part 1120. The second connecting component is selectively connected to the first connecting component. The first connecting component and/or the second connecting component are configured to be rotatable about the axis of the inner tube 2200, and the handle is configured such that, when the second connecting component is connected to the first connecting component, the first operating part 1120 is prevented from driving the inner tube 2200 to move axially, and when the second connecting component and/or the first connecting component rotate around the axis of the first operating part 1120 to cause the second connecting component and the first connecting component to be disconnected from each other, the first operating part 1120 is allowed to drive the inner tube 2200 to move in the direction from distal to proximal. That is to say, when the conveyor conveys the medical implant into the patient's body, the second connecting component and the first connecting component remain connecting with each other first, and when the release of the medical implant is completed, the second connecting component and the first connecting component are disconnected from each other. Thereafter, the first operating part 1120 is operated to drive the inner tube 2200 to move back quickly to close the outer tube 2100 (that is, the distal end of the inner tube 2200 returns into the outer tube 2100), thereby reducing adverse effects on the patient. In one non-limiting implementation, the second connecting component is rotatable about the axis of the inner tube 2200.

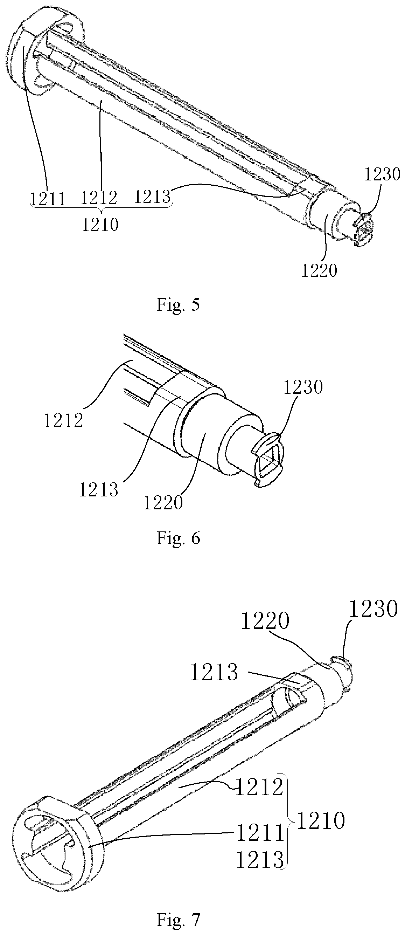

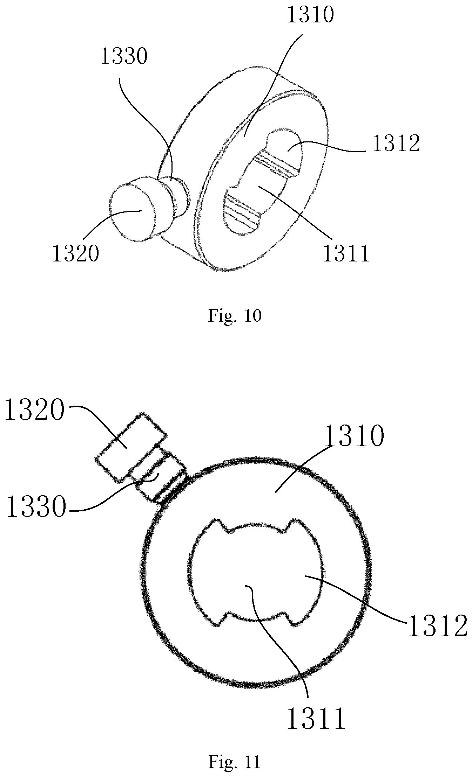

In more detail, referring to Fig. 3 in conjunction with Figs. 4 to 11, the first operating part 1120 may be disposed proximally to the housing 1110. When the first operating part 1120 is driven (manually or electrically in practice) to move away from the housing 1110, the inner tube 2200 is driven to move back. The first connecting component includes a connector tube 1220 and a first limiting part 1230. The connector tube 1220 is connected to the housing 1110. Optionally, the connector tube 1220 is connected to the housing 1110 by a first positioning seat 1210. Specifically, the first positioning seat 1210 is connected to the inner wall of the housing 1110, the connector tube 1220 is connected to the proximal end of the first positioning seat 1210, and the proximal end of the connector tube 1220 extends toward the first operating part 1120. The first limiting part 1230 is connected to the connector tube 1220, preferably to the proximal end of the connector tube 1220, and extends outward along the radial direction of the connector tube 1220 (as shown in Figs 5 to 8). Here, the first limiting part 1230 extends along the radial direction of the connector tube 1220, including the situation where the first limiting part 1230 extends strictly along the radial direction of the connector tube 1220, and the situation where the first limiting part 1230 extends substantially along the radial direction of the connector tube 1220, as long as the first limiting part 1230 protrudes from the outer peripheral surface of the connector tube 1220. The proximal end of the inner tube 2200 extends toward proximally through the first positioning seat 1210 and the connector tube 1220 to directly or indirectly connect to the first operating part 1120 (the specific way of connection will be described later). The second connecting component includes a second positioning seat 1310. The second positioning seat 1310 is movably connected to the inner wall of the first operating part 1120, and is configured to rotatable around the axis of the first operating part 1120. The second positioning seat 1310 has therein a first central through hole 1311 and an avoidance slot 1312 (as shown in Figs. 10 and 11). The first central through hole 1311 is configured for allowing the proximal end of the connector tube 1220 to pass through. The avoidance slot 1312 is connected to the first central through hole 1311 and is configured for allowing the first limiting part 1230 to pass through.

When the first limiting part 1230 is located proximally to the second positioning seat 1310 and is staggered with the avoidance slot 1312 in the circumferential direction, and when the first limiting part 1230 is pressed against the proximal end face of the second positioning seat 1310, the second connecting component is connected to the first connecting component (as shown in Fig. 4). At this time, the first limiting part 1230 and the second positioning seat 1310 limit each other so that the first operating part 1120 is prevented from moving in the axial direction under the action of external force, thereby preventing the first operating part 1120 from driving the inner tube 2200 to move axially. When the second positioning seat 1310 rotates under the action of external force, until the avoidance slot 1312 aligns with the first limiting part 1230 in the circumferential direction and allows the first limiting part 1230 to pass through, the first connecting component and the second connecting component are disconnected from each other. At this time, the first limiting part 1230 and the second positioning seat 1310 are released from limiting each other, and no longer hinder the axial movement of the first operating part 1120. Therefore, the operator can pull back the first operating part 1120 and drive the inner tube 2200 to move back, and at the same time, the second positioning seat 1310 moves in a direction from distal to proximal, and the connector tube 1220 and the first limiting part 1230 pass through the second positioning seat 1310 at the same time to a side distal to the second positioning seat 1310.

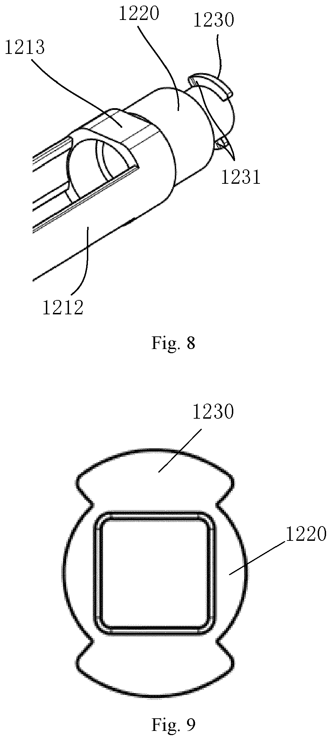

In the embodiment of the present invention, it is preferred that the shape and size of the avoidance slot 1312 match the shape and size of the first limiting part 1230. Taking Figs. 5 to 9 as an example, the first limiting part 1230 and the avoidance slot 1312 are both fan-shaped, and the avoidance slot 1312 is slightly larger than the first limiting part 1230. Therefore, the first limiting part 1230 is allowed to pass through the avoidance slot 1312. In other embodiments, the first limiting part 1230 may be square-shaped, special-shaped or of other shapes. Furthermore, please focus on Figs. 8 and 9, a guide surface 1231 is formed on the distal end of the first limiting part 1230, and the distance from the guide surface 1231 to the axis of the connector tube 1220 gradually increases along the direction from distal to proximal. Therefore, the first limiting part 1230 can pass through the avoidance slot 1312 smoothly.

The number of the first limiting parts 1230 is preferably multiple, for example, two or three. The multiple first limiting parts 1230 are evenly arranged along the circumference of the connector tube 1220 to improve the force on the second positioning seat 1310 to be evenly distributed when the first connecting component and the second connecting component are connected. Therefore, the service life is improved. The number of the avoidance slots 1312 is not less than the number of the first limiting parts 1230. Preferably, the number of the avoidance slots 1312 may be equal to the number of the first limiting parts 1230. The avoidance slots 1312 are arranged correspondingly to the first limiting parts 1230. For example, when the number of the first limiting parts 1230 is two, the number of the avoidance slots 1312 may also be two, and the two avoidance slots 1312 are arranged at equal intervals on the circumference of the first central through hole 1311. As those skilled in the art can understand, the angle that the second positioning seat 1310 rotates to switch the first connecting component and the second connecting component from a connected state to a non-connected state (where they are disconnected from each other) depends on the number and arrangement of the first limiting parts 1230. For example, when the number of the first limiting parts 1230 is two, and the two first limiting parts 1230 are evenly arranged along the circumference of the connector tube 1220, the second positioning seat 1310 can be rotated 90° in a predetermined direction to switch the first connecting component and the second connecting component from a connected state to a non-connected state.

To facilitate operation, the portion of the first operating part 1120 corresponding to the second positioning seat 1310 defines thereon a first slide slot 1121 extending circumferentially. The second connecting component also includes a first lever 1320. One end of the first lever 1320 is connected to the second positioning seat 1310, and the other end of the first lever 1320 extends out of the first operating part 1120 through the first slide slot 1121. In this way, the operator can use the first lever 1320 to exert an external force to the second positioning seat 1310 so as to move the first lever 1320 along the circumferential direction of the first operating part 1120 and to drive the second positioning seat 1310 to rotate around the axis of the first operating part 1120. The position and length of the first slide slot 1121 (that is, the size of the first slide slot 1121 in the circumferential direction of the first operating part 1120) depend on the number and arrangement of the first limiting parts 1230. Preferably, when the first connecting component is connected to the second connecting component, the first lever 1320 is located at one end of the first slide slot 1121; when the first connecting component is disconnected from the second connecting component, the first lever 1320 is located at the other end of the first slide slot 1121. This makes it easy to determine whether the second positioning seat 1310 is rotated in place.

Further, the second connecting component further includes a first elastic sleeve 1330. The first elastic sleeve 1330 is sleeved on the first lever 1320, and the outer wall of the first elastic sleeve 1330 is in contact with the wall of the first slide slot 1121, so that the first elastic sleeve 1330 is deformed. In other words, there is a mutual extrusion force between the first elastic sleeve 1330 and the wall of the first slide slot 1121. When the operator applies an external force to the first lever 1320 to move the first lever 1320 along the circumferential direction of the second operating part 1130, resistance is generated between the first elastic sleeve 1330 and the wall of the first slide slot 1121. On one hand, this resistance can enhance the operator's feel, and on another hand, the operator needs to provide a certain amount of external force to move the first lever 1320, which avoids misoperation. The resistance of the first lever 1320 when moving can be adjusted by adjusting the size of the first elastic sleeve 1330, and the first elastic sleeve 1330 can be directly replaced after wear, resulting in a convenience in maintenance.

In a non-limiting embodiment, the outer tube 2100 is used as a second movable tube, and the handle 1000 may further include a second operating part 1130 for connecting with the outer tube 2100 so as to drive the outer tube 2100 to move in the direction from distal to proximal. Specifically, after the conveyor conveys the medical implant into the patient's body, the inner tube 2100 remains stationary, and the outer tube 2100 is driven to move back by the second operating part 1130 to complete the release of the medical implant. Here, it is preferable that the second operating part 1130 is rotatably connected to the proximal end of the housing 1110, and the first operating part 1120 is arranged proximally to the second operating part 1130. In order to prevent the connection between the second operating part 1130 and the outer tube 2100 (the way of connection between the two will be described later) from being interfered by the first connecting component, the first positioning seat 1210 includes a third positioning sub-seat 1211, a connecting arm 1212 and a second positioning sub-seat 1213 that are connected. The first positioning sub-seat 1211 is connected to the inner wall of the housing 1110. The number of the connecting arms 1212 is at least two, preferably two, and the two ends of each the connecting arm 1212 are respectively connected with the first positioning sub-seat 1211 and the second positioning sub-seat 1213, and the second positioning sub-seat 1213 is also connected to the connector tube 1220. It can be understood that both the first positioning sub-seat 1211 and the second positioning sub-seat 1213 have channels for the catheter assembly to pass through. The two connecting arms 1212 are evenly arranged on the circumference of the first positioning sub-seat 1211 to make the first connecting component more stable, and the gap between the two connecting arms 1212 provides enough space for the connection between the outer tube 2100 and the second operating part 1130 (the specific way of connection will be described later).

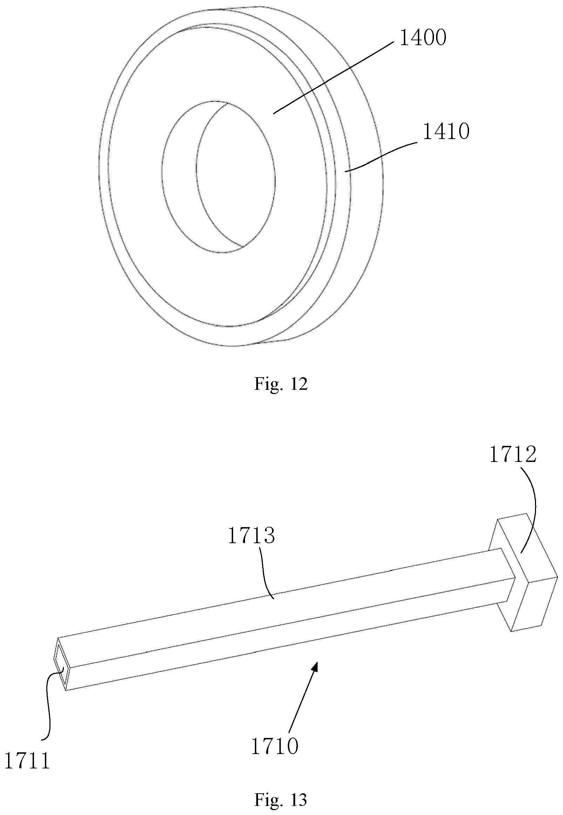

Preferably, the area of the distal end face of the connector tube 1220 is smaller than the area of the proximal end face of the second positioning sub-seat 1213. The handle further includes an auxiliary connecting plate 1400. As shown in Fig. 12, the shape of the auxiliary connecting plate 1400 is preferably circular, and a circular second slide slot 1410 may be defined in the distal end face of the auxiliary connecting plate 1400. The auxiliary connecting plate 1400 is used to be sleeved on the connector tube 1220, and the distal end of the auxiliary connecting plate 1400 is pressed against the proximal end face of the second positioning sub-seat 1213. At the same time, the proximal end of the second operating part 1130 abuts against the second slide slot 1410 and can rotate along the second slide slot 1410 under the action of external force. When the second connecting component is connected to the first connecting component, the distal end of the second operating part 1130 is pressed against the proximal end face of the auxiliary connecting plate 1400. Of course, the proximal end face of the auxiliary connecting plate 1400 may be provided with an annular groove that matches the distal end of the second operating part 1130.

Please refer back to Fig. 3, the handle 1000 also includes a third connecting component. The third connecting component is disposed in the housing 1110. The third connecting component is drivingly connected to the second operating part 1130, and is also used to connect with the proximal end of the outer tube 2100 (that is, the outer tube 2100 is indirectly connected to the second operating part 1130 through the third connecting component).

In more detail, the third connecting component may include a screw rod 1510. The distal end of the screw rod 1510 is connected to the proximal end of the outer tube 2100, and the proximal outer surface of the screw rod 1510 is provided with threads. The inner wall of the second operating part 1130 is provided with threads engaging with the threads on the proximal end of the screw rod 1510 for spiral transmission. Alternatively, the third connecting component includes a screw rod 1510 and a threaded sleeve 1520. The outer wall of the threaded sleeve 1520 is connected to the inner wall of the second operating part 1130, and the threaded sleeve 1520 is configured to rotate synchronously with the second operating part 1130. The inner wall of the threaded sleeve 1520 is provided with threads. The distal end of the screw rod 1510 is connected to the proximal end of the outer tube 2100. The outer surface of the proximal end of the screw rod 1510 is provided with threads engaging with the threads on the threaded sleeve 1520 for spiral transmission. In this way, when the second operating part 1130 rotates, the screw rod 1510 can be driven to move in the axial direction of the housing, and thereby the outer tube 2100 can be driven to move in the axial direction. It can be understood that the threads on the screw rod 1510 engage with the threads on the second operating part 1130 or the threaded sleeve 1520 at the gap between the two connecting arms 1212.

Optionally, an observation window 1112 may also be provided on the housing 1110 for observing the axial movement of the screw rod 1510. A scale mark can be provided on the screw rod 1510 to facilitate the operator to observe the axial movement distance of the screw rod 1510 and thereby know the axial movement of the outer tube 2100.

In actual work, when the conveyor conveys certain medical implants into place and releases them, it is required to prevent the outer tube 2100 from further moving back at a specific release position in order to perform other operations. In order to facilitate the operator to accurately perform the release operation, the handle 1000 also includes an axial limiting assembly, which is partially disposed in the housing and includes a first limiting structure 1610 and a second limiting structure 1620. The first limiting structure 1610 is connected to the housing 1110 and is configured to be rotatable around the axis of the housing 1110. The second limiting structure 1620 is connected to the third connecting component, specifically connected to the screw rod 1510, and preferably connected to the distal end of the screw rod 1510. The handle 1000 is configured such that when the outer tube 2100 moves a predetermined distance from distal to proximal, the first limiting structure 1610 and the second limiting structure 1620 engage with each other so that the outer tube 2100 is prevented from further moving from distal to proximal, when the first limiting structure 1610 rotates around the axis of the housing 1110 and disengages from the second limiting structure 1620, the outer tube is allowed to further moving along the direction from distal to proximal.

In a non-limiting embodiment, the first limiting structure 1610 includes a third positioning seat 1611 and a second limiting part 1612. The third positioning seat 1611 is movably connected to the housing 1110 and is configured to rotate around the axis of the housing 1110. The second limiting part 1612 is connected to the third positioning seat 1611 and at least partially protrudes from the distal end face of the third positioning seat 1611, and the second limiting part 1612 is also configured to be rotatable synchronously with the third positioning seat 1611. The second limiting structure 1620 includes a third limiting part 1621 protruding from the outer peripheral surface of the screw rod 1510.

When the conveyor has not started to release the medical implant, the third limiting part 1621 is located distally to the second limiting part 1612 and is axially spaced apart from the second limiting part 1612 by a predetermined distance, and the third limiting part 1621 also aligns with the second limiting part 1612 in the circumferential direction. In this way, in the initial stage of the release of the medical implant, when the operator drives the outer tube 2100 to move the predetermined distance in the direction from the distal to proximal by using the second operating part 1130, the third limiting part 1621 abuts against the second limiting part 1612 which blocks the outer tube 2100 from further moving in the direction from distal to proximal. At this time, other operations can be performed, such as adjusting the position and posture of medical implants. Thereafter, the operator rotates the third positioning seat 1611 so that the second limiting part 1612 is staggered with the third limiting part 1621 in the circumferential direction, and the second limiting structure 1620 and the third limiting part 1621 are disengaged with each other. Therefore, the second operating part 1130 is allowed to drive the outer tube 2100 to further move in the direction from distal to proximal.

Further, the housing 1110 has thereon a third slide slot 1111 extending axially. The first limiting structure 1610 also includes a second lever 1613. One end of the second lever 1613 is connected to the third positioning seat 1611, and the other end of the second lever 1613 extends out of the housing 1110 through the third slide slot 1111, so that it is convenient for the operator to operate. Preferably, the first limiting structure 1610 also includes a second elastic sleeve 1614. The second elastic sleeve 1614 is sleeved on the second lever 1613, and the outer wall of the second elastic sleeve 1614 is in contact with the wall of the third slide slot 1111 so that the second elastic sleeve 1614 deforms. That is to say, there is resistance between the second elastic sleeve 1614 and the wall of the third slide slot 1111 when the second elastic sleeve 1614 is moving, which enhances the operator's feel and also reduces the possibility of misoperation. The length and specific position of the third slide slot 1111 in the circumferential direction of the housing 1110 depend on the circumferential dimensions of the second limiting part 1612 and the third limiting part 1621. Generally, when the third limiting part 1612 and the third limiting part 1621 abut against each other, the second lever 1613 is located at one end of the third slide slot 1111, and when the second lever 1613 moves to the other end of the third slide slot 1111, the second limiting part 1612 and the third limiting part 1621 are circumferentially staggered with each other.

Not only that, the second limiting part 1612 is movably connected to the third positioning seat 1611 and is configured to movable along the axial direction of the housing to change in length the portion of the second limiting part 1612 protruding from the distal end face of the third positioning seat 1611, thereby changing the distance between the second limiting part 1612 and the third limiting part 1621 before the medical implant is released. The range for application of the conveyor is increased. The predetermined distance is adjusted according to the specific type and size of the medical implant when the delivery device is assembled. In an optional implementation, the third positioning seat 1611 is provided with a screw hole, the axis of the screw hole is parallel to the axis of the housing, and the second limiting part 1612 is composed of a screw rod passing through the screw hole on the third positioning seat 1611.

In addition, in the embodiment of the present invention, it is preferred that the third positioning seat 1611 has a circular ring structure, which can be formed by two semi-circular ring structures connected together to facilitate assembly. The proximal end of the screw rod 1510 of the third connecting component passes through the third positioning seat 1611 and then threadably engages with the second operating part 1130 or the threaded sleeve 1520.

Please refer back to Fig. 4, the handle 1000 further includes a fourth connecting component. The fourth connecting component is connected to the inner wall of the first operating part 1120 and is also used to connect to the proximal end of the inner tube 2200. Optionally, the fourth connecting component includes an anti-rotation member 1710 having a second central through hole 1711 for allowing the inner tube 2200 to pass through (as shown in Fig. 13). The anti-rotation member 1710 includes a first anti-rotation part 1712 and a second anti-rotation part 1713. The first anti-rotation part 1712 is connected to the inner wall of the first operating part 1120 and is configured to remain stationary relative to the first operating part 1120 in the circumferential direction, and the second anti-rotation part 1713 is at least partially inserted into the inner cavity of the connector tube 1220 and configured to remain stationary relative to the connector tube 1220 in the circumferential direction. Specifically, the first anti-rotation part 1712 and the second anti-rotation part 1713 each have a non-rotational shape. For example, the cross sections of the first anti-rotation part 1712 and the second anti-rotation part 1713 are rectangle. The shape of the cross section of at least part of the inner cavity of the connector tube 1220 matches the shape of the second anti-rotation part 1713, that is, the cross section of at least part of the inner cavity of the connector tube 1220 is also rectangle (as shown in Fig. 9). Therefore, the connector tube 1220 can remain stationary relative to the second anti-rotation part 1713 in the circumferential direction. By providing the anti-rotation member 1710, the inner tube 2200 can be prevented from rotating when the operator pulls the first operating part 1120 to drive the inner tube 2200 to move in the direction from distal to proximal.

In addition, the proximal end of the inner tube 2200 passes through the first operating part 1120 and is connected with a Luer connector for connection with a syringe or other external equipment.

It should also be noted that, in order to facilitate the assembly of the conveyor, the housing 1110, the first operating part 1120 and the second operating part 1130 each have preferably an assembled structure. The housing 1110 includes a first sub-housing and a second sub-housing. The second operating part 1130 includes a third sub-housing and a fourth sub-housing. The first operating part 1120 includes a fifth sub-housing and a sixth sub-housing. In addition, the conveyor also includes a first fastening ring 1101 and a second fastening ring 1102 (as annotated in Fig. 1). The first fastening ring 1101 is provided between the housing 1110 and the second operating part 1130 to ensure a firm connection between them. The second fastening ring 1102 is provided at the proximal end of the first operating part 1120, so that the fifth sub-housing and the sixth sub-housing are firmly connected.

It should also be noted that, in the case where the outer tube is used as the first movable tube, the connector tube may be connected to the distal side of the first positioning seat, and the first limiting part is preferably connected to the distal end of the connector tube. When the first connecting component is connected to the second connecting component, the first limiting part is located distally to the second positioning seat. Accordingly, the guide surface is provided at the proximal end of the first limiting part, and the distance between the guide surface and the axis of the connector tube gradually increases in the direction from proximal to distal.

Furthermore, the embodiment of the present invention also provides a handle, which is the handle 1000 as mentioned above.

Furthermore, the embodiment of the present invention also provides a medical device, including a medical implant and the aforementioned conveyor. The medical implant is to be compressed between the inner tube 2200 and the outer tube 2100.

Although the present invention is disclosed above, it is not limited thereto. Various changes and modifications can be made to the present invention by those skilled in the art without departing from the spirit and scope of the invention. Accordingly, the invention is intended to embrace all such modifications and variations if they fall within the scope of the appended claims and equivalents thereof.

## Claims

1. A handle, for controlling a catheter assembly, the catheter assembly comprising an outer tube and an inner tube partially inserted in the outer tube, the outer tube or the inner tube used a first movable tube; wherein the handle comprises:
a housing;
a first operating part, connected to the housing, and configured to drive the first movable tube to move in a first direction;
a first connecting component connected in the housing; and
a second connecting component connected to the first operating part, and the second connecting component selectively connected to or disconnected from the first connecting component;
wherein the first connecting component and/or the second connecting component are configured to be rotatable about an axis of the first movable tube; and the handle is configured such that, when the second connecting component and the first connecting component are connected with each other, the first operating part is prevented from driving the first movable tube to move in the first direction, when the first connecting component and/or the second connecting component rotate around the axis of the first movable tube to cause the first connecting component and the second connecting component to disconnect from each other, the first operating part is allowed to drive the first movable tube to move in the first direction.

2. The handle according to claim 1, wherein the inner tube is used as the first movable tube, and the first direction is a direction from distal to proximal.

3. The handle according to claim 1 or 2, wherein the first connecting component comprises a connector tube and a first limiting part, wherein the connector tube is connected to the housing and is configured for allowing the first movable tube to pass through; the first limiting part is connected to the connector tube and extends outward along a radial direction of the connector tube;
wherein the second connecting component comprises a second positioning seat, wherein the second positioning seat is connected to an inner wall of the first operating part and is configured to be rotatable around an axis of the first operating part; the second positioning seat defines therein a first central through hole and an avoidance slot, the avoidance slot is connected with the first central through hole;
wherein the handle is configured such that, when the connector tube passes through the first central through hole, and when the first limiting part is staggered with the avoidance slot in a circumferential direction and pressed against the second positioning seat, the second connecting component and the first connecting component are connected to each other, when the second positioning seat rotates until the avoidance slot aligns with the first limiting part in the circumferential direction and allows the first limiting part to pass through, the second connecting component and the first connecting component are disconnected from each other.

4. The handle according to claim 3, wherein a guide surface is formed on the first limiting part, a distance between the guide surface and an axis of the connector tube gradually increases along the first direction, and the guide surface is configured for guidance when the first limiting part passes through the avoidance slot.

5. The handle according to claim 3, wherein the first operating part defines thereon a first slide slot extending along a circumferential direction of the first operating part; the second connecting component further comprises a first lever having one end connected to the second positioning seat and a further end extending out of the first operating part through the first slide slot.

6. The handle according to claim 5, wherein the second connecting component further comprises a first elastic sleeve sleeved on the first lever, and an outer wall of the first elastic sleeve is in contact with a wall of the first slide slot so that the first elastic sleeve deforms.

7. The handle according to claim 3, wherein the first connecting component further comprises a first positioning seat, and the connector tube is connected to the housing by the first positioning seat; the first positioning seat comprises a first positioning sub-seat, a connecting arm and a second positioning sub-seat that are sequentially connected along the first direction; the first positioning sub-seat is connected to the housing; the second positioning sub-seat is connected to the connector tube.

8. The handle according to claim 7, wherein a number of the connecting arms is at least two, and the at least two connecting arms are arranged at intervals along a circumference of the first positioning sub-seat.

9. The handle according to claim 7, wherein the first operating part is arranged proximally to the housing;
the handle further comprises an auxiliary connecting plate that is sleeved on the connector tube and pressed against a proximal end face of the second positioning sub-seat;
when the first connecting component and the second connecting component are connected with each other, a distal end of the first operating part is pressed against a proximal end face of the auxiliary connecting plate.

10. The handle according to claim 3, wherein when the inner tube is used as the first movable tube, the handle further comprises a fourth connecting component that is connected to the inner wall of the first operating part and also configured to connect with a proximal end of the inner tube.

11. The handle according to claim 10, wherein the fourth connecting component comprises an anti-rotation member having a second central through hole for allowing the inner tube to pass through; the anti-rotation member comprises a first anti-rotation part and a second anti-rotation part; the first anti-rotation part is connected to the first operating part and is configured to remain circumferentially stationary relative to the first operating part; the second anti-rotation part is at least partially inserted into an inner cavity of the connector tube and is configured to remain circumferentially stationary relative to the connector tube.

12. A conveyor, comprising a catheter assembly and the handle according to any one of claims 1 to 11, wherein the catheter assembly comprises an outer tube and an inner tube partially inserted in the outer tube, and the outer tube or the inner tube is used a first movable tube; the first operating part of the handle is connected to the first movable tube and is configured to drive the first movable tube to move along the first direction.

13. A medical device, comprising the conveyor of claim 12 and a medical implant, wherein the medical implant is configured to be compressed between the inner tube and the outer tube.
